# FASCICULE DE BREVET EUROPEEN

(11) **EP 3 283 147 B1**
(45) Date de publication et mention de la délivrance du brevet: **06.02.2019**
(21) Numéro de dépôt: 16723415.2
(22) Date de dépôt: 15.04.2016
(51) Int. Cl.: A61M 11/00, A61B 5/00, A61G 10/02, A61M 16/14

(54) **UNITE MOBILE OU TRANSPORTABLE D'EXPOSITION AUX ALLERGENES**
MOBILE ODER TRANSPORTIERBARE EINHEIT ZUR EXPOSITION GEGENÜBER ALLERGENEN
MOBILE OR TRANSPORTABLE UNIT FOR EXPOSURE TO ALLERGENS

(30) Priorité: 16.04.2015 FR 1553384
(43) Date de publication de la demande: 21.02.2018
(73) Titulaire: Alyatec, 67000 Strasbourg (FR)
(72) Inventeur: SANTAILLER, Gérard, 69480 Marcy (FR)
(74) Mandataire: Merckling, Norbert
(86) Numéro de dépôt international: PCT/FR2016/050884
(87) Numéro de publication internationale: WO 2016/166491

(56) Documents cités:
- WO-A2-2013/163517
- JP-A- 2005 230 035
- JP-A- 2006 218 380
- JP-A- 2010 063 967
- JP-A- 2011 025 727
- JP-A- 2011 041 765
- US-A1- 2009 145 466
- C. LIDÈN ET AL: "A New Whole-body Exposure Chamber for Human Skin and Lung Challenge Experiments-the Generation of Wheat Flour Aerosols", ANNALS OF OCCUPATIONAL HYGIENE, vol. 42, no. 8, 1 novembre 1998 (1998-11-01), pages 541-547, XP055134038, ISSN: 0003-4878, DOI: 10.1093/annhyg/42.8.541 cité dans la demande
- W. EDUARD ET AL: "Generation and Homogeneity of Aerosols in a Human Whole-Body Inhalation Chamber", ANNALS OF OCCUPATIONAL HYGIENE, vol. 52, no. 6, 7 juillet 2008 (2008-07-07), pages 545-554, XP055134071, ISSN: 0003-4878, DOI: 10.1093/annhyg/men039 cité dans la demande
- PIYUSH PATEL ET AL: "Mobile Natural Exposure Chamber Technology Standardizes Controlled Environmental Exposure Chamber Challenges Across Multicenter National and International Allergy Trials", JOURNAL OF ALLERGY AND CLINICAL IMMUNOLOGY, vol. 131, no. 2, 1 février 2013 (2013-02-01), page AB188, XP055240751, AMSTERDAM, NL ISSN: 0091-6749, DOI: 10.1016/j.jaci.2012.12.1339 cité dans la demande
- H NANDKESHORE ET AL: "Validation of a unique mobile environmental exposure chamber system for use in multiple locations in a multicenter allergy trial demonstrates accuracy and reproducibility in grass pollen aerosolization after mobile chamber disassembly, relocation and reassembly", ALLERGY, vol. 68, no. Issue Supplement s.97, 18 septembre 2013 (2013-09-18), page 193, XP055240826, United Kingdom ISSN: 0105-4538, DOI: 10.1111/all.12250 cité dans la demande
- ROENBORG S M ET AL: "EXPOSURE CHAMBER FOR ALLERGEN CHALLENGE THE DEVELOPMENT AND VALIDATION OF A NEW CONCEPT", ALLERGY, WILEY-BLACKWELL PUBLISHING LTD, UNITED KINGDOM, vol. 51, no. 2, 1 février 1996 (1996-02-01), pages 82-88, XP008007828, ISSN: 0105-4538
- CHRISTIAN MONSÉ ET AL: "Considerations for the design and technical setup of a human whole-body exposure chamber", INHALATION TOXICOLOGY, vol. 24, no. 2, 1 janvier 2012 (2012-01-01), pages 99-108, XP055134058, ISSN: 0895-8378, DOI: 10.3109/08958378.2011.640362 cité dans la demande

## Description

La présente invention concerne une unité, mobile ou transportable, comprenant un compartiment d'exposition aux allergènes destiné à accueillir un ou plusieurs patients et dans laquelle on réalise une atmosphère d'inhalation à teneur contrôlée en allergènes en vue d'une provocation allergique chez ces patients.

Le compartiment d'exposition aux allergènes est une enceinte confinée, de type dit EEC (appelée Environmental Exposure Chamber ou European Exposure Chamber), connue également sous le nom de chambre de test d'allergie.

Les allergies sont un fléau mondial qui concerne plus d'une personne sur quatre en Occident. La recherche médicale dans le domaine de l'allergie et notamment celle se rapportant au développement de médicaments antiallergiques ou de traitements de désensibilisation, est de ce fait un secteur qui se développe considérablement.

Pour réaliser des études cliniques relatives à l'allergie ou pour évaluer l'efficacité de nouveaux médicaments ou de traitement de désensibilisation, il est indispensable d'observer la réaction de patients allergiques lorsqu'ils se retrouvent exposés aux allergènes naturels.

La réalisation de telles observations en milieu naturel, de façon scientifique et objective, est très difficile en raison des importantes fluctuations de la quantité d'allergènes inhalés par un patient en fonction, par exemple, de la saison, des conditions climatiques ou des endroits fréquentés par le patient. En effet, la quantité d'allergènes naturellement présents dans l'air ambiant est très variable selon par exemple la région où l'on se trouve, la saison, les conditions météorologiques notamment la température, l'humidité ou la présence de vent, le moment de la journée, ou même la hauteur par rapport au niveau du sol.

Pour pouvoir s'affranchir de ces très nombreux paramètres qui fluctuent de façon difficilement contrôlable, on a développé dans l'art antérieur des dispositifs appelés chambres de test d'allergie ou EEC (European/Environmental Exposure Chamber).

Ces dispositifs permettent d'accueillir des patients dans un lieu clos où l'on diffuse une quantité contrôlée d'allergènes et d'observer scientifiquement leur réaction physiologique après un temps d'exposition plus ou moins long. Les différents paramètres expérimentaux au sein de la chambre doivent être constants et contrôlés tout au long de l'expérimentation. On peut ainsi réaliser des tests dans des conditions reproductibles sur plusieurs patients ou plusieurs fois sur un même patient et obtenir immédiatement et de façon fiable des résultats comparables les uns avec les autres.

On connaît ainsi par exemple la chambre de test d'allergie décrite dans le brevet européen n° EP 1335750 au nom de HORAK ou celles décrites dans les demandes de brevet FRAUNHOFER WO 2010/0063714 et PATEL WO 2007/140601 ou encore ITSUKI SANGYO JP 2010 063967.

Ces chambres de test d'allergie antérieures sont des systèmes à grande échelle prévues pour accueillir simultanément un grand nombre de patients. Elles nécessitent des installations importantes et sont toutes réalisées dans des bâtiments ou structures fixes, par exemple au sein d'un centre médical ou d'un hôpital.

En outre, ces dispositifs antérieurs connus ne permettent pas d'obtenir une concentration en allergènes homogène et constante dans toute la salle d'exposition.

En effet, dans les premiers dispositifs, le mélange entre l'air et les particules d'allergènes se fait directement dans la salle d'exposition au moyen d'un fort courant d'air turbulent ou de ventilateurs.

Dans le dispositif japonais ITSUKI SANGYO, la substance est envoyée dans la salle d'exposition à travers une multitude d'ouvertures situées dans le plafond de celle-ci, puis est aspirée hors de la salle d'exposition à travers une multitude d'ouvertures situées dans le plancher de celle-ci. La substance aspirée retourne vers le dispositif d'injection pour être de nouveau envoyée dans la salle d'exposition, le dispositif fonctionnant en circuit fermé. Avec un tel recyclage de la substance injectée, il est impossible de garantir une concentration constante dans le temps car la quantité de substance présente dans les rejets n'est pas maîtrisable.

En outre, le mélange est également réalisé au moyen d'un ventilateur qui se trouve à la sortie du dispositif d'injection qui n'est pas un nébuliseur et est parfaitement incompatible avec une composition aqueuse d'allergènes. Une substance aussi fragile que des allergènes ne pourrait passer à travers les pales d'un ventilateur sans risquer d'être fortement endommagée.

On connait également les systèmes d'exposition décrits dans les publications suivantes : W. EDUARD ET AL. « Generation and Homogeneity of Aérosol in a Human Whole-Body Inhalation Chamber », C. LINDEN ET AL. "A New Whole-body Exposure Chamber for Human Skin and Lung Challenge Experiments - the Generation of Wheat Flour Aérosol" et CHRISTIAN MONSE ET AL. "Considérations for the design and technical setup of a human whole-body exposure chamber".

Le mélange obtenu avec ces dispositifs antérieurs n'est pas non plus satisfaisant car le mélange avec l'air de ventilation se fait directement dans le conduit d'arrivée d'air, que ce soit au moyen d'un conduit intérieur coaxial débouchant longitudinalement dans le conduit d'air comme dans le premier document, ou au moyen d'une simple connexion en T comme dans les deux autres documents. Or, le flux d'air circule trop rapidement dans le conduit d'arrivée d'air pour qu'un véritable mélange homogène se produise. Les particules sont simplement aspirées par effet Venturi et entraînées sans mélange réel entre les différents flux.

En outre, il s'agit là encore de systèmes fixes qui nécessitent des installations importantes et qui ne sont pas autonomes.

Et même si LINDEN et al. précisent que leur système pourrait idéalement être démantelé, pour pouvoir être déplacé et réassemblé, il ne s'agit que d'un déménagement ponctuel de ce dispositif d'un lieu fixe à un autre, et non d'un système réellement mobile. Ces opérations de démantèlement et de remontage sont longues et compliquées et impliquent forcément des phases de requalification importantes après le déplacement et le remontage.

Par ailleurs, lorsque l'on fait fonctionner ces systèmes connus de grande taille pour une session d'exposition à un allergène qui dure généralement plusieurs heures, la quantité d'allergène consommée est nécessairement importante si l'on veut obtenir une concentration en allergènes homogène dans toute la salle d'exposition et constante dans le temps. Les frais induits par cette consommation d'allergènes, en plus de tous les autres frais de fonctionnement, sont conséquents.

Il est donc essentiel d'essayer d'optimiser au mieux le remplissage de ces salles d'exposition afin d'éviter de multiplier les séances. Il est en effet très peu rentable de faire fonctionner ces dispositifs avec un nombre de patients inférieur à la capacité maximale de celle-ci.

Or, il n'est pas facile de recruter un nombre important de patients allergiques, présentant le profil médical précis recherché, qui soient volontaires pour participer à une expérimentation médicale et être exposés à un allergène dans une telle salle d'exposition.

Ces systèmes étant localisés de manière permanente dans un lieu géographique donné, les patients susceptibles d'être volontaires sont limités à ceux habitant à proximité de l'installation.

Si certains patients peuvent accepter de participer à une ou plusieurs expérimentions, d'autres n'auront pas forcément envie de renouveler cette expérience plusieurs fois ou trop fréquemment. De plus, ils ne sont pas toujours tous disponibles en même temps.

En outre, si l'on souhaite réaliser successivement plusieurs études scientifiques, un renouvellement des patients volontaires exposés, est obligatoire et indispensable sur un plan éthique, médical et scientifique, afin d'éviter pour ces patients des prises de médicaments à tester trop fréquentes. Ces médicaments sont en effet susceptibles d'interagir les uns avec les autres, ce qui pourrait être dangereux pour la santé des patients et/ou pourrait fausser les résultats expérimentaux.

De plus, lorsque l'on souhaite réaliser une étude avec un allergène peu ou non présent dans la région dans laquelle la salle d'exposition est implantée, peu de patients allergiques à cet allergène sont connus localement. Même si certaines personnes à terrain favorable le sont probablement, elles n'en sont généralement pas conscientes.

Toutes ces raisons font qu'il est souvent difficile de recruter suffisamment de patients volontaires pour utiliser régulièrement ces systèmes d'exposition fixes à leur pleine capacité. Pour réaliser une étude scientifique probante, les sessions avec une salle d'exposition peu remplie sont alors multipliées afin d'obtenir un nombre suffisant de patients pour compléter l'étude, ce qui augmente considérablement le coût des études réalisées.

L'invention permet de pallier ce désavantage, car elle propose une unité qui est mobile ou transportable, de petite taille, et qui peut avantageusement aller directement au contact des patients.

Les auteurs (PATEL et al.) des articles : « Mobile Natural Exposure Chamber Technology Standardizes Controlled Environmental Exposure Chamber Challenges Across Multicenter National and International Allergy Trials » et « Validation of a unique mobile environmental exposure chamber system for use in multiple locations in a multicenter allergy trial demonstrates accuracy and reproducibility in grass pollen aerosolization after mobile chamber disassembly, relocation and reassembly » tentent d'apporter une solution à ce problème en proposant un dispositif à enceinte extérieure gonflable destiné à être déplacé dans différents lieu.

Cependant, ce dispositif n'est toujours pas satisfaisant car, prévu pour cinquante personnes, il continue à être de grande taille et donc à présenter les mêmes inconvénients en termes de remplissage et de coût de fonctionnement que les chambres fixes.

En outre, pour être transporté, ce système doit être préalablement démonté. Une fois arrivé sur place, il doit être déployé et réinstallé avant toute utilisation. Or si le gonflage de l'enceinte peut être effectué en quelques heures, l'assemblage de tous les autres éléments fonctionnels, ainsi que les opérations de requalification du système, indispensables après un tel démontage et remontage, nécessitent plusieurs jours.

Ce système est donc loin d'être utilisable immédiatement après son arrivée sur site. Comme, en plus, les durées d'exposition aux allergènes et d'observations consécutives des patients nécessitent chacune plusieurs heures, l'immobilisation forcée de ce système est donc particulièrement longue.

En outre, pour son fonctionnement, ce système antérieur doit être raccordé à des installations existantes de fourniture et de traitement d'air, telles que celles d'un hôpital par exemple, et n'est pas autonome.

L'unité selon l'invention est au contraire mobile ou transportable en l'état, c'est-à-dire à l'état assemblée et en état de fonctionnement. Elle peut donc être utilisée quasi-immédiatement après son arrivée sur site, sans qu'il soit nécessaire de procéder à de longues phases de qualification préalables comme dans l'art antérieur.

Sa durée d'immobilisation étant considérablement réduite, il parfaitement envisageable de déplacer chaque jour l'unité selon l'invention et de procéder à une session d'exposition quotidienne à chaque fois dans un lieu différent.

Cette unité peut ainsi être envoyée dans des zones géographiques beaucoup plus étendues et/ou plus éloignées, afin d'aller à la rencontre de patients qui sans cela ne se seraient pas déplacés jusqu'à la salle d'exposition fixe.

Elle peut également être envoyée dans une zone géographique éloignée où l'allergène à tester est largement présent et dans laquelle des patients allergiques volontaires sont connus.

Le nombre de patients potentiels pouvant être testés est ainsi considérablement augmenté.

De plus, du fait de sa petite taille, son fonctionnement est très économique

En effet, l'unité selon l'invention comporte un compartiment d'exposition de petite taille qui peut accueillir un nombre limité de patients, un ou deux par exemple, et pas plus de quatre simultanément. Il est donc beaucoup plus facile de trouver suffisamment de volontaires pour la remplir à chaque session d'exposition, d'autant plus que l'unité peut être déplacée entre chaque session pour aller à la rencontre de nouveaux volontaires.

Le coût nécessaire à une session d'exposition reste limité du fait de la quantité d'allergène beaucoup plus faible à utiliser. Le nombre de session d'exposition peut donc être multiplié sans problème, tout en garantissant que la capacité maximale de remplissage soit atteinte à chaque fois. Le coût global d'une étude menée au moyen d'une telle unité est donc beaucoup plus faible.

Comme elle est autonome, l'unité selon l'invention peut en outre être envoyée et utilisée dans n'importe quel site, sans condition d'équipement sur place. Elle peut fonctionner de manière satisfaisante dans un endroit quelconque faiblement équipé, tout en garantissant des conditions expérimentales contrôlées (température, pression et humidité notamment) et une homogénéité spatiale et temporelle optimale de la concentration en allergènes dans le compartiment d'exposition.

L'unité selon l'invention offre ainsi avantageusement une très grande flexibilité, une rapidité, une simplicité et une souplesse d'utilisation jamais égalées jusqu'à présent, tout en fournissant des résultats parfaitement fiables d'un point de vue scientifique.

D'autre part, l'unité mobile ou transportable peut également être utilisée avantageusement pour présélectionner parmi les patients allergiques volontaires, ceux dont la réponse à une exposition est la plus adaptée pour chaque étude particulière à mener.

En effet, tous les patients allergiques ne réagissent pas de la même façon lorsqu'ils sont exposés à un allergène. Certains patients présentent une réaction immédiate, alors que d'autres présentent une réponse retardée après l'exposition ou encore les deux types de réponse. La réponse biologique, immunologique et clinique, ainsi que l'intensité de cette réponse peut varier d'un patient à l'autre. Toutes ces données médicales personnelles de réponse, sont spécifiques à chacun des patients et traduisent leur façon personnelle de réagir en cas d'exposition à un allergène.

Dans le cadre de cette demande de brevet, on appellera données médicales personnelles de réponse, les données médicales (biologiques, immunologiques et/ou cliniques) observables et/ou mesurables chez un patient pendant ou à la suite d'une exposition à un allergène, qui sont variables selon le patient et traduisent sa façon personnelle de répondre à cette exposition allergénique.

Selon l'étude à réaliser, et notamment selon la nature du médicament antiallergique à tester, il peut être intéressant ou plus efficace d'observer les effets d'une exposition allergénique chez une catégorie particulière prédéfinie de patients (par exemple les patients qui présentent une réponse immédiate ou retardée, ou ceux dont les symptômes sont très importants, ou dont les symptômes réapparaissent rapidement...).

Pour éviter de réaliser des tests coûteux et inutiles sur des patients qui ne réagissent pas de façon appropriée par rapport à l'objectif de l'étude à mener, il est nécessaire de sélectionner parmi l'ensemble des patients allergiques volontaires ceux dont les données médicales personnelles de réponse à l'exposition allergénique correspondent aux valeurs recherchées.

Pour être capable de faire cette présélection, il faut préalablement recenser l'ensemble des patients allergiques volontaires et déterminer leurs données médicales personnelles de réponse lors d'une exposition à l'allergène étudié, afin de pouvoir les classer selon la valeur de ces données.

L'unité mobile ou transportable de l'invention est parfaitement adaptée pour réaliser une telle classification, également appelée « screening ».

L'invention enseigne en effet l'utilisation d'une unité d'exposition aux allergènes mobile ou transportable conforme à l'invention, pour :
- aller à proximité du ou des lieux où se trouvent des patients allergiques à un allergène;
- soumettre ces patients à une exposition contrôlée audit allergène au sein de l'unité d'exposition aux allergènes, mobile ou transportable ;
- observer ou mesurer au moins une donnée médicale personnelle de réponse pour chacun de ces patients ;
et de préférence également pour :
- enregistrer dans une base de données cette donnée médicale personnelle de réponse pour chacun de ces patients.

Cette base de données peut être avantageusement interrogeable pour sélectionner parmi cet ensemble de patients, une catégorie particulière de patients en fonction des valeurs de leur donnée médicale personnelle de réponse.

Ladite donnée médicale personnelle de réponse peut être choisie par exemple, de manière non exhaustive, parmi : la vitesse d'apparition des symptômes, la durée de manifestation des symptômes, la dose d'allergènes nécessaire pour provoquer l'apparition d'un symptôme, la nature ou l'intensité des symptômes, la concentration en immunoglobuline, la variation du VEMS (Volume Expiratoire Maximal par Seconde) ou du DEP (Débit Expiratoire de Pointe) ou de la CVF (Capacité Vitale Forcée) pendant l'exposition, le temps nécessaire pour atteindre une diminution prédéfinie (par exemple 60%) du VEMS ou du DEP ou de la CVF.

Selon un mode de réalisation préférentiel de l'invention, l'unité mobile ou transportable est utilisée en complément d'un système fixe d'exposition aux allergènes et seuls les patients appartenant à la catégorie particulière sélectionnée sont ensuite appelés à se déplacer jusqu'au système fixe d'exposition aux allergènes dans lequel ils sont soumis à une exposition contrôlée audit allergène.

L'unité mobile ou transportable selon l'invention peut ainsi être utilisée en complément d'une chambre d'exposition fixe classique. Elle permet avantageusement de présélectionner les patients qui seront ensuite envoyés dans la chambre fixe.

Le remplissage de la chambre fixe est donc optimisé en ne recrutant que des patients qui présentent les critères réactionnels souhaités. Les conditions de fonctionnement (durée, concentration...) de la chambre fixe peuvent être adaptées de façon optimale aux patients présents. L'efficacité de l'étude scientifique et le caractère probant de ses résultats s'en trouvent améliorés, tout en régulant les coûts de fonctionnement.

En outre, on évite de faire venir, parfois de loin, des gens ne convenant pas à l'étude qui se retrouveraient écartés par la suite. On économise ainsi des frais importants correspondant à leurs indemnisations et à leurs frais de transport et d'hébergement, et on évite de décourager les patients volontaires qui, sollicités inutilement, pourraient par la suite refuser de participer à une étude ultérieure.

Pour résoudre le problème technique, l'invention enseigne ainsi une unité d'exposition aux allergènes destinée à accueillir un ou plusieurs patients venant inhaler de l'air chargé en particules d'allergènes en vue d'une provocation allergique.

Selon l'invention, cette unité est mobile ou transportable à l'état assemblé et fonctionnel. Elle est autonome et utilisable immédiatement après transport, sans phase de qualification préalable.

Cette unité comprend :
- un compartiment d'exposition confiné, contenant de l'air chargé en particules d'allergènes et destiné à accueillir entre un et quatre patients en vue d'une provocation allergique ;
- un sas par lequel les patients entrent dans le compartiment d'exposition ou sortent du compartiment d'exposition ;
- un dispositif d'injection d'allergènes de type nébuliseur qui produit un flux de particules d'allergènes à partir d'une composition aqueuse d'allergènes ;
- un système de traitement d'air qui aspire de l'air extérieur et l'envoie après traitement sous la forme d'un flux d'air dépourvu d'allergènes dans un conduit d'arrivée d'air ; et
- une chambre de mélange, distincte et séparée du compartiment d'exposition, du dispositif d'injection d'allergènes et du conduit d'arrivée d'air, mais communiquant avec ceux-ci et formant un élargissement par rapport au conduit d'arrivée d'air de manière à provoquer une expansion du flux d'air dépourvu d'allergènes lorsqu'il pénètre dans la chambre de mélange, chambre de mélange dans laquelle se produit un mélange entre le flux de particules d'allergènes provenant du dispositif d'injection d'allergènes et le flux d'air dépourvu d'allergènes amené par le conduit d'arrivée d'air, et qui communique avec le compartiment d'exposition de manière à envoyer dans celui-ci l'air chargé en particules d'allergènes résultant du mélange.

Selon les modes de réalisation, l'unité d'exposition aux allergènes selon l'invention peut être réalisée dans un camion, une remorque, une semi-remorque, un bus aménagé ou un conteneur transportable.

Selon un mode de réalisation préférentiel de l'invention, elle comporte en outre un moyen de mise à niveau et/ou un système de fixation à suspensions pour le dispositif d'injection d'allergènes.

Selon un mode de réalisation de l'invention, cette unité comprend une zone technique, séparée du compartiment d'exposition, et dans laquelle se trouve le dispositif d'injection d'allergènes.

Lorsque l'unité d'exposition selon l'invention est réalisée dans un véhicule routier ou est prévue pour être transportée par un tel véhicule, sa zone technique est préférentiellement disposée à l'extrémité de l'unité située ou destinée à être placée du côté de la cabine de pilotage dudit véhicule routier, car il s'agit généralement de l'endroit où les vibrations sont les plus faibles.

Selon les modes de réalisation de l'invention, la zone technique peut comprendre en outre un ou plusieurs des moyens suivants : le système de traitement d'air, un laboratoire de préparation de l'allergène, du matériel d'analyse biologique, un réfrigérateur ou un congélateur, des moyens de réglage ou de contrôle des paramètres d'exposition.

Selon un mode de réalisation de l'invention, l'unité mobile ou transportable comprend une zone médicale et d'accueil, séparée du compartiment d'exposition, et dans laquelle se trouve au moins un des moyens suivants : un fauteuil, un lit ou une table de consultation ; du matériel médical ; un chariot d'urgence ; un dispositif de mesure d'une donnée médicale personnelle de réponse des patients ; un spiromètre ; une armoire à pharmacie ; un réfrigérateur ou un congélateur ; un bureau ; une chaise ; du matériel informatique, administratif ou de bureau ; un laboratoire de préparation de l'allergène ; du matériel d'analyse biologique ; des dispositifs de mesure ou de contrôle des paramètres d'exposition.

Selon un mode de réalisation de l'invention, le système de traitement d'air comprend un ou plusieurs moyens choisis parmi des moyens de filtration de l'air, des moyens de chauffage de l'air, des moyens de refroidissement de l'air, des moyens de régulation de l'humidité de l'air, des moyens de réglage du débit de l'air.

Selon une variante préférentielle de l'invention, le compartiment d'exposition peut comporter une colonne de soufflage, centrale, qui communique avec la chambre de mélange et délivre l'air chargé en particules d'allergènes.

Selon un mode de réalisation de l'invention, l'unité mobile ou transportable peut comprendre deux sas distincts, l'un par lequel les patients entrent dans le compartiment d'exposition et l'autre par lequel les patients sortent du compartiment d'exposition.

Selon un mode de réalisation de l'invention, l'unité mobile ou transportable peut comporter en outre une zone d'hygiène contenant un WC et un point d'eau.

Selon une variante préférentielle de l'invention, elle peut comprendre en outre une cuve de récupération des effluents liquides, et/ou un conteneur de récupération des déchets solides, et/ou un conteneur de récupération des déchets médicaux de type DASRI.

D'autres caractéristiques et avantages de l'invention apparaitront à la lecture de la description détaillée qui va suivre, description faite en référence aux dessins annexés, dans lesquels :
- la figure 1 est une vue de dessus schématique d'un premier exemple d'unité mobile selon l'invention ;
- la figure 2 est une vue de dessus schématique d'un deuxième exemple d'unité mobile selon l'invention ;
- la figure 3 est une vue de dessus schématique d'un troisième exemple d'unité mobile selon l'invention ;
- la figure 4 est une vue de dessus schématique d'un quatrième exemple d'unité mobile selon l'invention.

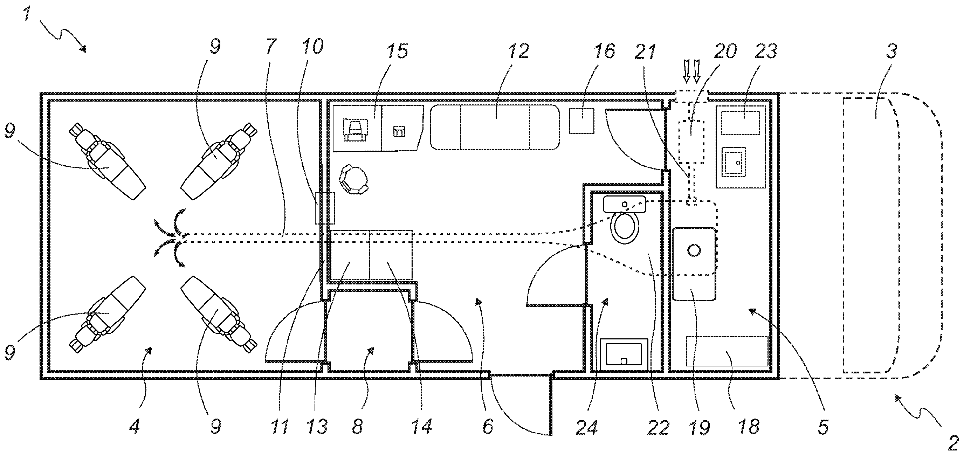

Le système d'exposition aux allergènes selon la présente invention va maintenant être décrit de façon détaillée en référence aux figures 1 à 4. Les éléments équivalents représentés sur les différentes figures porteront les mêmes références numériques.

On a représenté sur ces figures plusieurs variantes d'une unité 1 d'exposition aux allergènes selon l'invention, de taille et d'aménagement intérieur varié.

Ces exemples d'unité 1 d'exposition ont tous été représentés comme étant réalisés dans ou montés sur un véhicule routier 2 dont la cabine de pilotage 3 est schématisée en traits discontinus. Cependant, il peut également s'agir d'une unité d'exposition réalisée dans un conteneur indépendant, par exemple iso, et transportable par un véhicule de nature quelconque et notamment routier, ferroviaire ou maritime.

Les unités 1 représentées comportent toutes un compartiment d'exposition 4, une zone technique 5 et une zone médicale et d'accueil 6.

Le compartiment d'exposition 4 est une pièce confinée qui est destinée à accueillir les patients durant l'exposition et dans laquelle on peut produire une atmosphère d'inhalation à teneur contrôlée en allergènes.

Pour cela, pendant les périodes d'exposition, de l'air chargé en particules d'allergènes est envoyé dans le compartiment d'exposition 4 par exemple au moyen d'une colonne de soufflage 7, de préférence centrale et préférentiellement située au plafond. Cet air est ensuite évacué vers l'extérieur au moyen de bouches d'évacuation non représentées préférentiellement disposées en partie basse du compartiment, et de préférence après filtration.

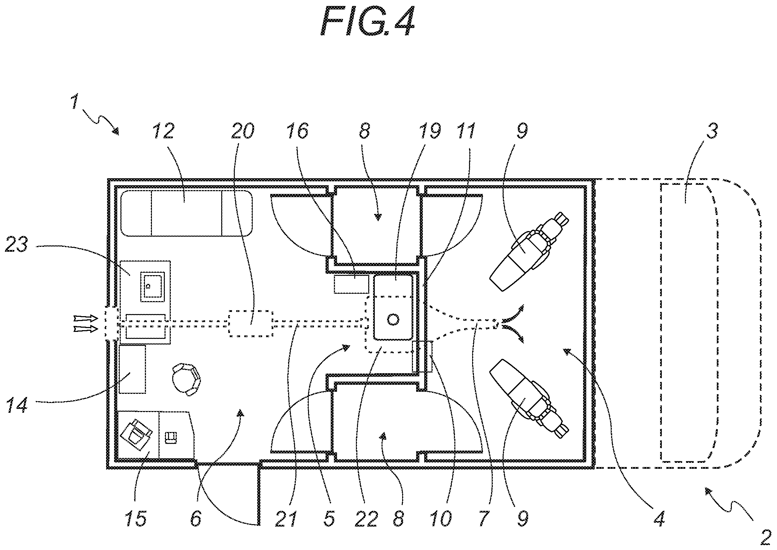

Le compartiment d'exposition 4 est accessible par l'intermédiaire d'un sas 8 qui sert d'interface entre le compartiment d'exposition 4 et la zone médicale et d'accueil 6. Selon les modes de réalisation, l'unité 1 peut comporter un unique sas 8 servant à la fois à l'entrée et à la sortie des patients, comme représenté sur les figures 1 à 3, ou bien deux sas 8 distincts servant pour l'un à l'entrée et pour l'autre à la sortie des patients comme représenté sur la figure 4.

Le compartiment d'exposition 4 contient, selon les variantes et la taille de l'unité 1, entre un et quatre fauteuils 9 dans lesquels les patients peuvent s'installer lors de l'exposition. Pour des raisons d'optimisation du fonctionnement de l'unité en utilisation, ces fauteuils sont préférentiellement au nombre de deux.

Les fauteuils 9 sont de préférence inclinables et sont généralement fixés au sol pour ne pas se déplacer pendant le transport de l'unité 1 qui est mobile ou transportable à l'état assemblé et fonctionnel. Selon une variante envisageable, ces fauteuils 9 sont décrochables et sont alors avantageusement mobiles afin de pouvoir éventuellement servir de brancard d'évacuation dans le cas où l'un des patients devrait être évacué d'urgence.

Selon les variantes d'aménagement de l'unité 1 d'exposition selon l'invention, le compartiment d'exposition 4 peut renfermer d'autres dispositifs et/ou matériels de nature variée, pas forcément représentés sur les figures.

Il peut ainsi par exemple contenir un dispositif de mesure 10 d'une ou plusieurs données médicales personnelles de réponse des patients présents dans le compartiment d'exposition 4, notamment un dispositif de mesure respiratoire. Il s'agit de préférence d'un dispositif de type spiromètre permettant de mesurer la capacité pulmonaire des patients.

Il peut comporter également un dispositif de communication, par exemple de type interphone ou numérique, permettant au patient de communiquer avec une personne qui surveille le bon déroulement de l'exposition et qui se trouve préférentiellement dans la zone médicale et d'accueil 6, ainsi qu'une ou plusieurs caméras facilitant cette surveillance.

Afin de permettre une meilleure surveillance des patients pendant la période d'exposition, les portes du ou des sas 8, ainsi que la paroi 11 séparant le compartiment d'exposition 4 de la zone médicale et d'accueil 6 sont préférentiellement entièrement ou au moins partiellement transparentes.

Du matériel médical classique, ainsi qu'un chariot d'urgence (si la zone médicale et d'accueil 6 n'en contient pas) peuvent également être prévus dans le compartiment d'exposition 4.

La zone médicale et d'accueil 6 est séparée du compartiment d'exposition 4 et permet d'accueillir le patient avant qu'il ne soit exposé à l'allergène afin de compléter son dossier administratif et de lui faire subir un examen médical approprié (pouvant par exemple consister en un interrogatoire médical, un examen clinique, des tests cutanés et/ou des prélèvements sanguins).

Elle permet également de garder le patient en observation pendant un certain temps après son exposition à un allergène.

Pour cela, elle contient une table de consultation 12 sur lequel le patient peut être examiné ou soigné en cas de besoin. Comme l'ensemble du mobilier de l'unité 1, la table de consultation est préférentiellement fixée au sol ou à la paroi pour ne pas bouger lors du transport. Elle peut cependant être prévue décrochable et mobile, afin de pouvoir avantageusement servir de brancard d'évacuation en cas d'urgence.

En fonction des besoins et de la place disponible, la zone médicale et d'accueil 6 peut comporter en outre : un placard ou une armoire 13 contenant par exemple des médicaments et/ou du matériel médical nécessaire aux soins ou à la phase de consultation préalable, un réfrigérateur/congélateur 14 permettant de conserver les échantillons prélevés, un bureau 15, une chaise, du matériel administratif, informatique ou de bureau (ordinateur, imprimante, dossiers patients...) permettant notamment la gestion administrative des patients et l'enregistrement des paramètres et résultats de l'exposition, un chariot d'urgence 16 standard contenant le matériel nécessaire à la prise en charge d'une urgence vitale, du matériel d'analyse biologique permettant par exemple de réaliser des tests ELISA ou de traiter les échantillons prélevés sur les patients, ou tout autre dispositif ou matériel approprié.

Comme mentionné précédemment, la zone médicale et d'accueil 6 peut comporter également un dispositif de communication avec le compartiment d'exposition 4 et/ou un dispositif de surveillance de celui-ci.

Elle peut également contenir un dispositif de mesure 10, notamment un dispositif de mesure respiratoire tel qu'un spiromètre par exemple, qui permet de mesurer une ou plusieurs données médicales personnelles des patients avant et/ou après leur exposition dans le compartiment d'exposition 4.

Pour éviter la multiplication des dispositifs, dans le cas où un même dispositif de mesure 10 doit être utilisé à la fois dans la zone médicale et d'accueil 6 et dans le compartiment d'exposition 4, il est possible de prévoir un dispositif de mesure 10 commun, placé à l'interface entre les deux zones et pouvant être utilisé par le patient des deux côtés de la paroi.

Un exemple d'un tel dispositif de mesure 10 commun été représenté sur les figures 1, 3 et 4. Il s'agit de préférence d'un spiromètre qui comporte un tuyau et un embout de chaque côté de la paroi 11.

L'unité 1 d'exposition aux allergènes selon l'invention comprend de préférence également un ou plusieurs dispositifs de récupération des déchets lui permettant de ne laisser aucun déchet dans les endroits où elle est envoyée.

Elle comprend ainsi par exemple une cuve de récupération des effluents liquides (non représentée) qui s'étend de préférence sous l'unité 1.

Elle peut également comprendre un conteneur de récupération des déchets médicaux 17 de type poubelle DASRI (Déchets d'Activités de Soin à Risques Infectieux) et un conteneur de récupération des déchets solides 18 dans lequel on jette par exemple les combinaisons et sur-chaussures jetables portés par les patients durant l'exposition, les embouts de spiromètre, les lingettes de nettoyage... Ces conteneurs sont préférentiellement situés dans la zone médicale et d'accueil 6 comme représenté sur la figure 6, mais ils peuvent être placés à tout autre endroit approprié et de place suffisante de l'unité 1.

Afin de produire l'air chargé en particules d'allergènes nécessaire à l'exposition des patients dans le compartiment d'exposition 4, l'unité 1 comprend un dispositif d'injection d'allergènes 19 qui produit un flux de particules d'allergènes à partir d'un échantillon d'allergènes.

Ce dispositif d'injection d'allergènes 19 est du type nébuliseur et fonctionne à partir d'un échantillon liquide d'allergènes. Selon un mode de réalisation préférentiel de l'invention, le dispositif d'injection d'allergènes 19 est un atomiseur par ondes capillaires.

Comme l'unité est mobile ou transportable à l'état assemblé et fonctionnel, et que le dispositif d'injection d'allergènes 19 est un dispositif assez fragile, sensible aux vibrations et qui pourrait se dérégler pendant le transport de l'unité 1 selon l'invention, il est préférable de prendre des mesures de précaution appropriées pour assurer une fixation adaptée pendant le transport et un fonctionnement optimal de celui-ci une fois l'unité 1 arrivée sur son lieu d'accueil. On peut ainsi prévoir par exemple, pour le dispositif d'injection d'allergènes 19, un système de fixation à suspensions et un moyen de mise à niveau avant utilisation.

Le dispositif d'injection 19 est ainsi utilisable immédiatement après transport, sans phase de qualification préalable. Seuls quelques petits réglages rapides peuvent être nécessaires.

Pour les mêmes raisons, lorsque l'unité 1 est transportée par un véhicule routier, ce dispositif d'injection d'allergènes 19 est préférentiellement placé à l'avant de l'unité 1, c'est-à-dire à proximité de l'extrémité de l'unité 1 dirigée vers la cabine de pilotage du véhicule routier, car il s'agit généralement de l'endroit où les vibrations sont les moins importantes pendant le roulage.

L'unité 1 comprend également un système de traitement d'air 20 qui aspire l'air extérieur et l'envoie après traitement sous la forme d'un flux d'air dépourvu d'allergènes dans un conduit d'arrivée d'air 21.

Le système de traitement d'air 20 comprend tous les moyens nécessaires pour transformer l'air extérieur aspiré en un air dépourvu d'allergènes présentant tous les paramètres appropriés pour réaliser l'exposition des patients dans des conditions satisfaisantes sur le plan scientifique. Il comprend ainsi notamment des moyens de filtration de l'air, des moyens de chauffage de l'air, des moyens de refroidissement de l'air, des moyens de régulation de l'humidité de l'air et/ou des moyens de réglage du débit de l'air.

L'unité 1 est ainsi parfaitement autonome et n'a donc pas besoin d'être raccordée au système de conditionnement d'air ou de ventilation d'un bâtiment préexistant, tel qu'un hôpital par exemple, comme c'est le cas avec les dispositifs de l'art antérieur.

L'unité 1 comprend enfin une chambre de mélange 22, dans laquelle se produit le mélange entre le flux de particules d'allergènes provenant du dispositif d'injection d'allergènes 19 et l'air dépourvu d'allergènes amené par le conduit d'arrivée d'air 21, et qui communique avec le compartiment d'exposition 4, via la colonne de soufflage 7 sur les exemples représentés, afin d'alimenter le compartiment d'exposition 4 en air chargé de particules d'allergènes résultant du mélange.

Cette chambre de mélange 22 forme un caisson creux, distinct du compartiment d'exposition 4, du dispositif d'injection d'allergènes 19 et du conduit d'arrivée d'air 21, et qui constitue un élargissement par rapport à ce dernier.

Cet élargissement provoque une expansion du flux d'air lorsqu'il pénètre dans la chambre de mélange 22. De ce fait, la vitesse de l'air est ralentie et il ne se crée que des miro-turbulences d'intensité réduite. On obtient ainsi un mélange particulièrement efficace du flux d'air avec le flux des particules d'allergènes qui ont le temps de s'interpénétrer et de se mélanger intimement avant d'arriver au niveau de la sortie vers le compartiment d'exposition 4, ce qui garantit une parfaite homogénéisation de la concentration d'allergènes dans l'ensemble du compartiment d'exposition 4.

Ce mélange est également très doux, ce qui limite le risque d'endommagement des particules fragiles d'allergènes et ne provoque que très peu de réarrangements des particules par agrégation, qui étaient fréquemment rencontrés avec les dispositifs antérieurs à brassages ou turbulences importantes. La taille des particules libérées dans le compartiment d'exposition 4 est beaucoup plus stable et homogène et peut ainsi être mieux contrôlée.

Selon une variante préférentielle, la section de la chambre de mélange 22 peut préférentiellement diminuer de manière à former un rétrécissement à proximité de la sortie vers le compartiment d'exposition 4. Le flux d'air chargé en particules d'allergènes est ainsi accéléré par compression juste avant sa sortie de la chambre de mélange 22 afin de pénétrer dans le compartiment d'exposition 4 avec une vitesse suffisante pour garantir le portage des particules d'allergènes dans la totalité du compartiment.

Tous ces dispositifs techniques peuvent se trouver à un endroit quelconque de l'unité 1, mais sont préférentiellement situés dans une zone technique 5, séparée du compartiment d'exposition 4.

Selon les variantes, cette zone technique 5 peut faire partie de la zone médicale et d'accueil 6 comme dans l'exemple de la figure 4, mais elle constitue plus préférentiellement une autre zone indépendante, distincte de la zone médicale et d'accueil 6 comme sur les figures 1 à 3.

La chambre de mélange 22 est préférentiellement réalisée dans le plafond de l'unité 1 mobile ou transportable selon l'invention, de préférence partiellement au-dessus de la zone technique 5 et du compartiment d'exposition 4.

Outre le dispositif d'injection d'allergènes 19 et le système de traitement d'air 20, la zone technique 5 peut selon les besoins comporter d'autres équipements, par exemple des moyens de réglage et de contrôle des paramètres d'exposition, un petit laboratoire 23 de préparation des échantillons d'allergènes, du matériel d'analyse biologique permettant par exemple de réaliser des tests ELISA ou de traiter les échantillons prélevés sur les patients, un réfrigérateur 14 et/ou un congélateur, un conteneur à déchets 18, un placard ou une armoire 13 contenant du matériel quelconque, ou tout autre dispositif ou matériel approprié.

Selon les aménagements, certains de ces dispositifs tels que par exemple le réfrigérateur/congélateur 14, le conteneur à déchets 18, ou le laboratoire 23 peuvent indifféremment être placés dans la zone technique 5 ou dans la zone médicale et d'accueil 6.

Certains de ces dispositifs peuvent également être omis afin de limiter l'encombrement. C'est par exemple le cas du laboratoire 23 si les échantillons d'allergènes peuvent être préparés préalablement et emportés tous prêts dans l'unité 1 mobile ou transportable de l'invention, ou d'autres dispositifs si l'on peut utiliser des dispositifs extérieurs équivalents situés à proximité des lieux d'accueil de l'unité 1.

Une zone d'hygiène 24, contenant par exemple un WC et un lavabo, peut également être prévue dans les unités 1 de grande taille, telles que celle représentée sur la figure 1.

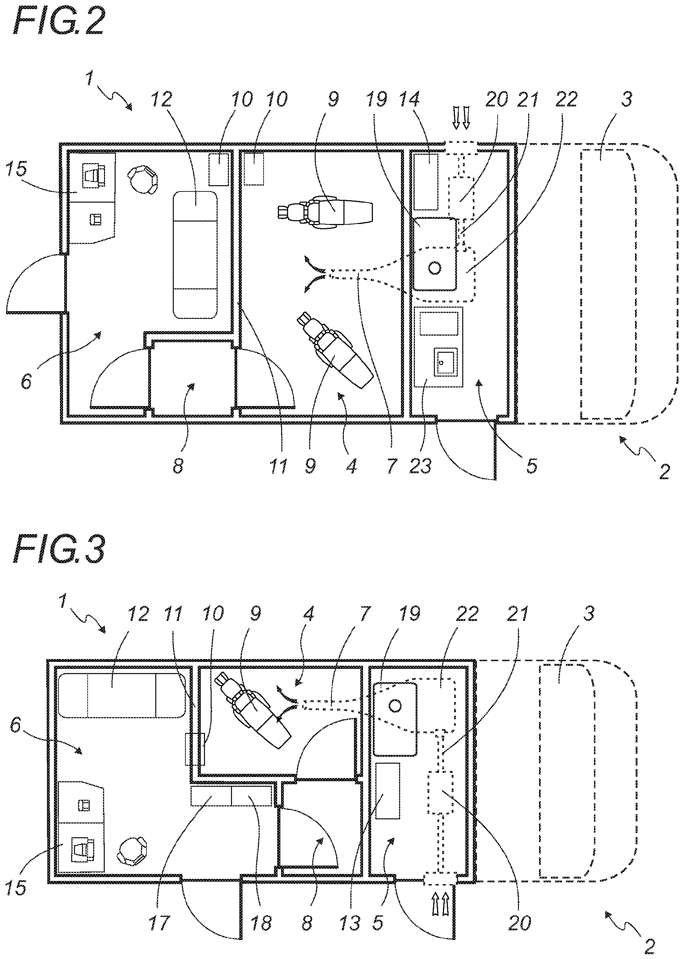

La figure 3 représente au contraire un exemple d'unité 1 d'exposition aux allergènes de taille réduite, qui peut être réalisée dans un véhicule de petite taille (un fourgon par exemple) pouvant ainsi avantageusement être conduit avec un permis de conduire classique.

Pour son fonctionnement, l'unité 1 mobile ou transportable selon l'invention
peut être conçue entièrement autonome afin de pouvoir être envoyée dans des zones complètement isolées.

En outre, la récupération des effluents liquides et de l'ensemble des déchets solides peut être organisée afin de ne générer aucune pollution notable.

Comme exposé dans la partie introductive, l'unité 1 mobile ou transportable selon l'invention peut être avantageusement utilisée pour mettre en oeuvre un procédé de classification de patients allergiques à un allergène, en fonction de leurs données médicales personnelles de réponse à une exposition à cet allergène, et alors que ces patients peuvent se trouver dans des lieux différents. En conséquence, elle peut avantageusement être utilisée pour créer et renseigner une base de données regroupant les informations relatives à ces patients allergiques et à leurs données médicales personnelles de réponse à une exposition à cet allergène.

De manière évidente, l'invention ne se limite pas aux modes de réalisation préférentiels décrits précédemment et représentés sur les différentes figures, l'homme du métier pouvant y apporter de nombreuses modifications et imaginer d'autres variantes sans sortir ni de la portée, ni du cadre de l'invention définis par les revendications.

## Revendications

1. Unité (1) d'exposition aux allergènes destinée à accueillir un ou plusieurs patients venant inhaler de l'air chargé en particules d'allergènes en vue d'une provocation allergique, unité (1) **caractérisée en ce qu'**elle est mobile ou transportable à l'état assemblé et fonctionnel, et est autonome et utilisable immédiatement après transport, sans phase de qualification préalable ; et
**en ce qu'**elle comprend :
- un compartiment d'exposition (4) confiné, contenant de l'air chargé en particules d'allergènes et destiné à accueillir entre un et quatre patients en vue d'une provocation allergique ;
- un sas (8) par lequel les patients entrent dans le compartiment d'exposition (4) ou sortent du compartiment d'exposition (4) ;
- un dispositif d'injection d'allergènes (19) de type nébuliseur qui produit un flux de particules d'allergènes à partir d'une composition aqueuse d'allergènes ;
- un système de traitement d'air (20) qui aspire de l'air extérieur et l'envoie après traitement sous la forme d'un flux d'air dépourvu d'allergènes dans un conduit d'arrivée d'air (21) ; et
- une chambre de mélange (22), distincte et séparée du compartiment d'exposition (4), du dispositif d'injection d'allergènes (19) et du conduit d'arrivée d'air (21), mais communiquant avec ceux-ci, et formant un élargissement par rapport au conduit d'arrivée d'air (21) de manière à provoquer une expansion du flux d'air dépourvu d'allergènes lorsqu'il pénètre dans la chambre de mélange (22), chambre de mélange (22) dans laquelle se produit un mélange entre le flux de particules d'allergènes provenant du dispositif d'injection d'allergènes (19) et le flux d'air dépourvu d'allergènes amené par le conduit d'arrivée d'air (21), et qui communique avec le compartiment d'exposition (4) de manière à envoyer dans celui-ci l'air chargé en particules d'allergènes résultant du mélange.

2. Unité (1) d'exposition aux allergènes selon la revendication 1 **caractérisé en ce qu'**elle est réalisée dans un camion, une remorque, une semi-remorque, un bus aménagé ou un conteneur transportable.

3. Unité (1) d'exposition aux allergènes selon l'une quelconque des revendications précédentes **caractérisée en ce qu'**elle comporte en outre un moyen de mise à niveau ou un système de fixation à suspensions pour le dispositif d'injection d'allergènes (19).

4. Unité (1) d'exposition aux allergènes selon l'une quelconque des revendications précédentes **caractérisée en ce qu'**elle comprend une zone technique (5), séparée du compartiment d'exposition (4), et dans laquelle se trouve le dispositif d'injection d'allergènes (19).

5. Unité (1) d'exposition aux allergènes selon la revendication précédente **caractérisée en ce qu'**elle est réalisée dans un véhicule routier (2) ou prévue pour être transportée par un véhicule routier (2), et **en ce que** la zone technique (5) est disposée à l'extrémité de l'unité (1) située ou destinée à être placée du côté de la cabine de pilotage (3) dudit véhicule routier (2).

6. Unité (1) d'exposition aux allergènes selon la revendication 4 **caractérisée en ce que** la zone technique (5) comprend en outre au moins un des moyens suivants : le système de traitement d'air (20), un laboratoire (23) de préparation de l'allergène, du matériel d'analyse biologique, un réfrigérateur (14) ou un congélateur, des moyens de réglage ou de contrôle des paramètres d'exposition.

7. Unité (1) d'exposition aux allergènes selon l'une quelconque des revendications précédentes **caractérisée en ce qu'**elle comprend une zone médicale et d'accueil (6), séparée du compartiment d'exposition, dans laquelle se trouve au moins un des moyens suivants : un fauteuil, un lit ou une table de consultation (12) ; du matériel médical ; un chariot d'urgence (16) ; un dispositif de mesure (10) d'une donnée médicale personnelle de réponse des patients, un spiromètre ; une armoire (13) à pharmacie ; un réfrigérateur (14) ou un congélateur ; un bureau (15) ; une chaise ; du matériel informatique, administratif ou de bureau ; un laboratoire (23) de préparation de l'allergène ; du matériel d'analyse biologique ; des dispositifs de mesure ou de contrôle des paramètres d'exposition.

8. Unité (1) d'exposition aux allergènes selon l'une quelconque des revendications précédentes **caractérisée en ce que** le système de traitement d'air (20) comprend un ou plusieurs moyens choisis parmi des moyens de filtration de l'air, des moyens de chauffage de l'air, des moyens de refroidissement de l'air, des moyens de régulation de l'humidité de l'air, des moyens de réglage du débit de l'air.

9. Unité (1) d'exposition aux allergènes selon l'une quelconque des revendications précédentes **caractérisée en ce que** le compartiment d'exposition (4) comporte une colonne de soufflage (7), centrale, qui communique avec la chambre de mélange (22) et délivre l'air chargé en particules d'allergènes.

10. Unité (1) d'exposition aux allergènes selon l'une quelconque des revendications précédentes **caractérisée en ce qu'**elle comprend deux sas (8) distincts, l'un par lequel les patients entrent dans le compartiment d'exposition (4) et l'autre par lequel les patients sortent du compartiment d'exposition (4).

11. Unité (1) d'exposition aux allergènes selon l'une quelconque des revendications précédentes **caractérisée en ce qu'**elle comporte en outre une zone d'hygiène (24) contenant un WC et un point d'eau, ou une cuve de récupération des effluents liquides, ou un conteneur de récupération des déchets solides (18), ou un conteneur de récupération des déchets médicaux (17).

12. Utilisation d'une unité (1) d'exposition aux allergènes mobile ou transportable selon l'une quelconque des revendications 1 à 11, pour :
- aller à proximité du ou des lieux où se trouvent des patients allergiques à un allergène;
- soumettre ces patients à une exposition contrôlée audit allergène au sein de l'unité (1) d'exposition aux allergènes, mobile ou transportable ;
- observer ou mesurer au moins une donnée médicale personnelle de réponse pour chacun de ces patients.

13. Utilisation d'une unité (1) d'exposition aux allergènes mobile ou transportable selon la revendication 12 et pour en outre :
- enregistrer dans une base de données cette donnée médicale personnelle de réponse pour chacun de ces patients.

14. Utilisation d'une unité (1) d'exposition aux allergènes selon la revendication 13 **caractérisée en ce que** la base de données est interrogeable pour sélectionner une catégorie particulière de patients en fonction de la valeur de leur donnée médicale personnelle de réponse.

15. Utilisation d'une unité (1) d'exposition aux allergènes selon l'une quelconque des revendications 12 à 14 **caractérisée en ce que** ladite donnée médicale personnelle de réponse est choisie parmi la vitesse d'apparition des symptômes, la durée de manifestation des symptômes, la dose d'allergènes nécessaire pour provoquer l'apparition d'un symptôme, la nature ou l'intensité des symptômes, la concentration en immunoglobuline, la variation du VEMS (Volume Expiratoire Maximal par Seconde) ou du DEP (Débit Expiratoire de Pointe) ou de la CVF (Capacité Vitale Forcée) pendant l'exposition, le temps nécessaire pour atteindre une diminution prédéfinie du VEMS ou du DEP ou de la CVF.

## Patentansprüche

1. Allergenexpositionseinheit (1) zur Aufnahme eines oder mehrerer Patienten, die sich darin befinden, um mit allergenen Partikeln belastete Luft einzuatmen, um eine allergische Reaktion zu provozieren, wobei die Einheit (1) **dadurch gekennzeichnet ist, dass** sie mobil und in zusammengebautem und einsatzbereitem Zustand transportiert werden kann und dass sie autonom und sofort nach dem Transport ohne vorherige Einstellungsphase einsatzbereit ist; und
dass sie umfasst:
- eine geschlossene Expositionskammer (4), die mit allergenen Partikeln belastete Luft enthält und zur Aufnahme von ein bis vier Patienten gedacht ist, um eine allergische Reaktion zu provozieren;
- eine Schleuse (8), durch die die Patienten die Expositionskammer (4) betreten oder die Expositionskammer (4) verlassen können;
- eine Vorrichtung zum Einblasen von Allergenen (19) vom Typ Zerstäuber, der einen Strom Allergenpartikeln, ausgehend von einer wässrigen Allergenlösung erzeugt;
- ein Luftaufbereitungssystem (20), das die Außenluft ansaugt und nach Behandlung in Form eines allergenfreien Luftstroms in eine Lufteinlassleitung (21) leitet; und
- eine Mischkammer (22), die von der Expositionskammer (4), der Allergen-Einblasvorrichtung (19) und der Lufteinlassleitung (21) abgetrennt ist, aber die mit diesen kommuniziert und welche, bezogen auf die Lufteinlassleitung (21), eine Erweiterung bildet, so dass es zu einer Ausdehnung der allergenfreien Luft kommt, wenn sie in die Mischkammer (22) gelangt, Mischkammer (22), in der der Allergenpartikelstrom aus der Allergen-Einblasvorrichtung (19) und der allergenfreie Luftstrom aus der Lufteinlassleitung (21) gemischt werden, und die mit der Expositionskammer (4) in Verbindung steht, so dass die aus der Mischung stammende, mit Allergenen belastete Luft in diese hinein geblasen werden kann.

2. Allergenexpositionseinheit (1) nach Anspruch 1, **dadurch gekennzeichnet, dass** sie aus einem Lastwagen, einem Anhänger, einem Auflieger, einem umgebauten Bus oder einem transportablen Container besteht.

3. Allergenexpositionseinheit (1) nach einem der vorangehenden Ansprüche, **dadurch gekennzeichnet, dass** sie außerdem über eine Nacheinstellmittel oder eine Befestigungssystem mit Aufhängungen für die Allergen-Einblasvorrichtung (19) enthält.

4. Allergenexpositionseinheit (1) nach einem der vorangehenden Ansprüche, **dadurch gekennzeichnet, dass** sie einen Technikbereich (5) enthält, die von der Expositionskammer (4) abgetrennt ist, in dem sich die Allergen-Einblasvorrichtung (19) befindet.

5. Allergenexpositionseinheit (1) nach dem vorhergehenden Anspruch, **dadurch gekennzeichnet, dass** sie als Straßenfahrzeug (2) ausgeführt ist oder für den Transport auf einem Straßenfahrzeug (2) vorgesehen ist, und dass der Technikbereich (5) am Ende der Einheit (1) angeordnet ist oder auf der Seite der Steuerkabine (3) dieses Straßenfahrzeugs (2) aufgestellt werden kann.

6. Allergenexpositionseinheit (1) nach Anspruch 4, **dadurch gekennzeichnet, dass** der Technikbereich (5) außerdem mindestens eine der folgenden Mitteln enthält: das Luftaufbereitungssystem (20), ein Labor (23) zur Allergenvorbereitung, biologisches Analysematerial, einen Kühlschrank (14) oder einen Gefrierschrank, Vorrichtungen zur Steuerung oder Kontrolle der Expositionsparameter.

7. Allergenexpositionseinheit (1) nach einem der vorangehenden Ansprüche, **dadurch gekennzeichnet, dass** sie einen Behandlungs- und Empfangsbereich (6) enthält, die von der Expositionskammer abgetrennt ist, in dem sich mindestens einer der vorliegenden Mittel befindet: ein Sessel oder eine Liege oder ein Tisch zur Untersuchung (12); medizinisches Material; ein Notfallwagen (16); eine Messvorrichtung (10) für individuelle Daten der Reaktion der Patienten, ein Spirometer; ein Medikamentenschrank (13); ein Kühlschrank (14) oder Gefrierschrank; ein Schreibtisch (15); ein Stuhl; EDV-, Verwaltungs- oder Büromaterial; ein Labor (23) zur Allergenvorbereitung; Vorrichtungen zur Steuerung oder Kontrolle der Expositionsparameter.

8. Allergenexpositionseinheit (1) nach einem der vorangehenden Ansprüche, **dadurch gekennzeichnet, dass** das Luftaufbereitungssystem (20) eine oder mehrere Mitteln enthält, die unter Mitteln zur Luftfilterung, Lufterwärmung, Luftkühlung sowie Vorrichtungen zur Regelung der Luftfeuchtigkeit und des Luftdurchsatzes ausgewählt wird/werden.

9. Allergenexpositionseinheit (1) nach einem der vorangehenden Ansprüche, **dadurch gekennzeichnet, dass** die Expositionskammer (4) eine zentrale Gebläsesäule (7) enthält, die mit der Mischkammer (22) in Verbindung steht und mit Allergenen belastete Luft einbläst.

10. Allergenexpositionseinheit (1) nach einem der vorangehenden Ansprüche, **dadurch gekennzeichnet, dass** sie zwei unterschiedliche Schleusen (8) enthält: eine, durch die die Patienten die Expositionskammer (4) betreten und eine weitere, durch die die Patienten die Expositionskammer (4) verlassen.

11. Allergenexpositionseinheit (1) nach einem der vorangehenden Ansprüche, **dadurch gekennzeichnet, dass** sie außerdem einen Hygienebereich (24) mit einem WC und einem Wasseranschluss, oder einem Becken zum Auffangen von Abwasser oder einen Behälter zum Aufnehmen fester Abfälle (18) oder einen Behälter zur Aufnahme medizinischer Abfälle (17) enthält.

12. Einsatz einer mobilen oder transportablen Allergenexpositionseinheit (1) nach einem der vorangehenden Ansprüche 1 bis 11 um:
- sich nahe an den Ort oder die Orte zu begeben, wo sich die gegenüber einem Allergen allergischen Patienten befinden;
- diese Patienten einer kontrollierten Exposition dieser Allergene innerhalb der mobilen oder transportablen Allergenexpositionseinheit (1) auszusetzen;
- mindestens einen individuellen persönlichen Reaktionswert für jeden dieser Patienten zu beobachten.

13. Einsatz einer mobilen oder transportablen Allergenexpositionseinheit (1) nach Anspruch 12 und, um außerdem:
- in einer Datenbank diesen persönlichen Reaktionswert für jeden dieser Patienten einzugeben.

14. Einsatz einer Allergenexpositionseinheit (1) nach Anspruch 13, **dadurch gekennzeichnet, dass** in der Datenbank die Abfrage nach einer besonderen Kategorie Patienten, entsprechend dem Wert ihres persönlichen Reaktionswertes möglich ist.

15. Einsatz einer Allergenexpositionseinheit (1) nach einem der vorangehenden Ansprüche 12 bis 14, **dadurch gekennzeichnet, dass** dieser persönliche Reaktionswert unter der Geschwindigkeit des Auftretens von Symptomen, der Dauer des Bestehens der Symptome, der erforderlichen Allergendosis, um das Auftreten eines Symptoms zu provozieren, der Art oder Intensität der Symptome, der Immunglobulinkonzentration, der FEV-1 (Einsekunden-Ausatemkapazität) oder dem Peak-Flow oder der FVC (forcierte exspiratorische Vitalkapazität) während der Exposition, der erforderlichen Zeit zum Erreichen einer vorher definierten Verringerung des FEV-1, Peak- Flow oder FVC ausgewählt wird.

## Claims

1. Allergen exposure unit (1) designed to accommodate one or more patients inhaling air charged with allergen particles in view of an allergenic provocation, with the said unit (1) being **characterized in that** it is mobile or transportable in its assembled and functional state, and it is autonomous and immediately usable after transportation, without any prior qualification phase, and **in that** it comprises:
▪ one confined exposure compartment (4), containing air charged with allergen particles, being designed to accommodate from one to four patients for an allergenic provocation;
▪ one airlock (8) through which the patients enter the exposure compartment (4) or exit the exposure compartment (4);
▪ one allergen injection device (19) of nebulizer type, that produces a flow of allergen particles from an aqueous composition of allergens;
▪ one air treatment system (20) that draws outside air and sends it after treatment in the form of an air flow free of allergens into an air inlet pipe (21); and
▪ one mixing chamber (22) that is distinct and separate from the exposure compartment (4), from the allergen injection device (19) and from the air inlet pipe (21), but that is connected to them, and that forms a widening with respect to the air inlet pipe (21), so as to cause an expansion of the allergen-free air flow when it enters the mixing chamber (22), mixing chamber (22) in which a mixture occurs between the flow of allergen particles coming from the allergen injection device (19) and the allergen-free air flow supplied by the air inlet pipe (21), and that is connected to the exposure compartment (4) so as to supply into it air charged with allergen particles resulting from the mixture.

2. Allergen exposure unit (1) according to claim 1, **characterized in that** it is realized in a truck, a trailer, a semi-trailer, an appropriately-equipped bus, or a transportable container.

3. Allergen exposure unit (1) according to any one of the preceding claims, **characterized in that** it also includes a leveling means or a suspended securing system for the allergen injection device (19).

4. Allergen exposure unit (1) according to any one of the preceding claims, **characterized in that** it comprises a technical area (5) that is separate from the exposure compartment (4), and in which the allergen injection device (19) is located.

5. Allergen exposure unit (1) according to the preceding claim, **characterized in that** it is realized in a road vehicle (2) or is designed to be transported by a road vehicle (2), and **in that** the technical area (5) is positioned at the extremity of the unit (1), which is located or intended to be positioned on the side of the driving cab (3) of the road vehicle (2).

6. Allergen exposure unit (1) according to claim 4, **characterized in that** the technical area (5) also comprises at least one of the following means: the air treatment system (20); one allergen preparation laboratory (23); biological analysis equipment; one refrigerator (14) or freezer; exposure parameter adjustment or monitoring means.

7. Allergen exposure unit (1) according to any one of the preceding claims, **characterized in that** it comprises a medical and reception area (6) that is separate from the exposure compartment, in which there is at least one of the following means: one armchair; one bed or consulting table (12); medical equipment; one crash cart (16); one measuring device (10) measuring a response personal medical data item of patients; one spirometer; one medicine cabinet (13); one refrigerator or freezer (14); one desk (15); one chair; computing, administrative or office equipment; one allergen preparation laboratory (23); biological analysis equipment; exposure parameter measurement or monitoring devices.

8. Allergen exposure unit (1) according to any one of the preceding claims, **characterized in that** the air treatment system (20) comprises one or more means selected from among: air filtering means; air heating means; air cooling means; air humidity regulation means; air flow rate regulation means.

9. Allergen exposure unit (1) according to any one of the preceding claims, **characterized in that** the exposure compartment (4) includes a central blow-out column (7) that is connected to the mixing chamber (22), and that supplies air charged with allergen particles.

10. Allergen exposure unit (1) according to any of the preceding claims, **characterized in that** it comprises two separate airlocks (8): one through which the patients enter the exposure compartment (4), and the other one through which patients exit the exposure compartment (4).

11. Allergen exposure unit (1) according to any one of the preceding claims, **characterized in that** it also includes a hygiene area (24) containing a WC and a water point, or a liquid effluent collector tank, or a solid effluent collection container (18), or a medical waste collection container (17).

12. Use of a mobile or transportable allergen exposure unit (1) according to any one of claims 1 to 11, to:
▪ go near the place or places where patients being allergic to an allergen are;
▪ submit these patients to a controlled exposure to the said allergen within the mobile or transportable allergen exposure unit (1);
▪ observe or measure at least one response personal medical data item for each of these patients.

13. Use of a mobile or transportable allergen exposure unit (1) according to claim 12, to additionally:
▪ record this response personal medical data item for each of these patients, in a database.

14. Use of an allergen exposure unit (1) according to claim 13, **characterized in that** the database can be queried to select a particular category of patients as a function of the value of their response personal medical data item.

15. Use of an allergen exposure unit (1) according to any one of claims 12 to 14, **characterized in that** the said response personal medical data item is selected from among: the speed of appearance of symptoms; the duration of persistence of symptoms; the dose of allergens necessary to cause the appearance of a symptom; the nature or intensity of the symptoms; the concentration of immunoglobulin; the variation of the Forced Expiratory Volume in One Second (FEV1) or the Peak Expiratory Flow (PEF) or the Forced Vital Capacity (FVC) during the exposure; the time necessary to attain a previously-defined decrease in the FEV1 or the PEF or the FVC.
